# FASCICULE DE BREVET EUROPEEN

(11) **EP 0 680 750 B1**
(45) Date de publication et mention de la délivrance du brevet: **15.12.1999**
(21) Numéro de dépôt: 95400966.8
(22) Date de dépôt: 27.04.1995
(51) Int. Cl.: A61K 7/48, A61K 7/00

(54) **Composition cosmétique ou pharmaceutique à usage topique**
Topisches kosmetisches oder pharmazeutisches Mittel
Topical cosmetic or pharmaceutic composition

(30) Priorité: 02.05.1994 FR 9405320
(43) Date de publication de la demande: 08.11.1995
(73) Titulaire: LABORATOIRES DE BIOLOGIE VEGETALE YVES ROCHER, 56200 La Gacilly (FR)
(72) Inventeur: Voultoury, Robert, F-92160 Antony (FR); Scott, Anne-Marie, F-60400 Noyon (FR)
(74) Mandataire: Le Guen, Gérard

(56) Documents cités:
- DE-C- 4 302 132
- FR-A- 2 684 388
- JOURNAL OF CELL BIOLOGY, vol.117, no.2, Avril 1992 pages 327 - 335 TZEN ET AL. 'SURFACE STRUCTURE AND PROPERTIES OF PLANT SEED OIL BODIES'

## Description

La présente invention concerne des compositions cosmétiques et des compositions pharmaceutiques à usage topique comprenant des vésicules lipidiques.

La présente invention concerne plus spécifiquement des compositions de ce type comprenant des vésicules lipidiques obtenues à partir des graines d'oléagineux.

Dans les graines d'oléagineux, l'huile (triglycérols essentiellement) est présente sous la forme de corps huileux d'un diamètre pouvant varier de 0,1 à 10 micromètres, et comprenant un noyau central d'huile entouré d'une enveloppe constituée de protéines (oléosines) et de phospholipides (J. TZEN et al., J. Cell. Bio, 117, 327, 1992).

Ces corps huileux peuvent être extraits des oléagineux d'origine par écrasement des graines d'oléagineux, émulsification dans une phase aqueuse et filtration.

La présente invention vise à utiliser ces corps huileux pour la fabrication de compositions cosmétiques et/ou pharmaceutiques à usage topique.

La présente invention a en conséquence pour objet une composition cosmétique ou pharmaceutique à usage topique comprenant des vésicules lipidiques obtenues par écrasement de graines d'oléagineux, émulsification dans une phase aqueuse et filtration.

Dans une forme avantageuse de l'invention, on incorpore dans les compositions, non pas l'émulsion telle qu'obtenue après filtration mais les vésicules lipidiques sous forme d'un concentrat (ayant l'aspect d'une crème) obtenu par centrifugation de l'émulsion.

En général, on peut incorporer dans une phase aqueuse le concentrat de vésicules lipidiques en des quantités pouvant représenter jusqu'à 30% en poids de la composition.

Comme exemples de graines d'oléagineux, on peut citer les graines de soja, pistache, macadamia, tournesol, colza, arachide, amande, noisette, sésame, bourrache, germe de blé, jojoba. On utilise de préférence des graines à forte teneur en huile (pistache, macadamia, arachide, jojoba, noisette).

La stabilisation des émulsions ou des concentrats peut être obtenue à l'aide notamment des techniques suivantes :

### stabilisation bactériologique

- traitement des graines par la chaleur (cuisson 1 heure ou plus à 50°C maximum) ;
- irradiation des graines de 0 à 10 kgray ;
- traitement UHT de l'émulsion de corps huileux (après filtration et avant la centrifugation) : 120°C x 5 secondes ;
- ajout éventuel, soit dans l'eau d'émulsification, soit dans la phase aqueuse, de dilution du concentrat, de conservateurs et par exemple :
   - . esters de l'acide parahydroxybenzoïque :: 0 à 5% en poids,
   - . Na₄ EDTA :: 0 à 0,1 % en poids
   - . acide sorbique :: 0 à 0,5 %
   - . imidazolidinylurée :: 0 à 0,5 % en poids
   - . déhydroacétate de sodium:: 0 à 0,5% en poids
   - . sulfite de sodium :: 0 à 0,5 % en poids

### Stabilisation physique des compositions

On peut parvenir, avec le minimum d'ajout de constituants chimiques à la phase aqueuse de dilution des concentrats, à une bonne stabilité des compositions finales par réglage du potentiel zéta des particules.

L'ajout de composants pertinents sera donc réglé grâce à cette technique.

Parmi ces composants, on peut citer :
- - chlorure de sodium: 0 à 2 % en poids
- - acide citrique: 0 à 2 % en poids
- - pectine hydrolysée: 0 à 1 % en poids
- - tampons phosphates: 0 à 1 % en poids

Ces compositions selon l'invention peuvent comprendre en outre des épaississants tels que :
- - acides polyacryliques: 0 à 1 % en poids
- - gomme xanthane: 0 à 1 % en poids
- - gomme sclérane: 0 à 1 % en poids
- - bentone et dérivés: 0 à 2 % en poids
- - polyacrylamide et dérivés: 0 à 2 % en poids
- - caroube et/ou caraghénannes: 0 à 2 % en poids

Les compositions selon l'invention peuvent en outre contenir des colorants et des parfums.

Ces compositions selon l'invention sont équivalentes à des compositions cosmétiques existantes, mais elles présentent vis-à-vis de celles-ci les avantages suivants :
- pas de système émulsionnant synthétique et exogène, d'où une meilleure tolérance cutanée ;
- les oléosines présentent des épitopes de configuration similaire à celles des apoprotéines des lipoprotéines animales. Elles peuvent donc être reconnues par les lipases cutanées.

Les compositions selon l'invention peuvent donc prétendre - par leur similitude biologique - à une meilleure substantivité que celle des compositions cosmétiques existantes basées sur des émulsionnants synthétiques.

On donnera ci-après des exemples illustrant l'invention.

### EXEMPLES D'APPLICATION

### EXEMPLE 1

### LAIT POUR LE VISAGE

On broie, au broyeur à couteaux, des graines de macadamia préalablement stabilisées au plan microbiologique.

On ajoute à 10 parties du broyat 90 parties d'eau additionnée ou non de conservateurs et antioxydants. On maintient une agitation moyenne pendant une demi-heure à 35°C maximum.

L'émulsion (lait) ci-dessus est alors filtrée en filtration frontale (cartouche 200 microns) avec dispositif de rinçage, de façon à éliminer la pulpe végétale.

L'émulsion filtrée de cette façon peut alors être concentrée par centrifugation. On préfère pour cette opération une machine de laiterie.

Le concentrat (crème) obtenu présente un pourcentage de matière grasse de l'ordre de 60% en poids. Ce concentrat peut alors être dilué, épaissi et parfumé pour former la composition suivante :
- - concentrat: 15 % à 30 % en poids
- - gomme xanthane: 1 à 2 % en poids
- - parfum: 0,1 à 0,5 % en poids
- - conservateurs: qs
- - eau: qs à 100 % en poids

### EXEMPLE 2

### PREPARATION D'UNE CREME DE NUIT

Le concentrat préparé à l'exemple 1 peut être utilisé pour fabriquer la composition suivante :
- - concentrat: 20 à 30 % en poids
- - glycérine: 2 à 5,0 % en poids
- - gomme xanthane: 1 à 2,0 % en poids
- - polyacrylamide: 5 à 10 % en poids
- - parfum: 0,1 à 0,5 % en poids
- - conservateurs: qs
- - eau: qs à 100 % en poids

## Revendications

1. Composition cosmétique ou pharmaceutique à usage topique comprenant des vésicules lipidiques obtenues par écrasement de graines d'oléagineux, émulsification dans une phase aqueuse et filtration.

2. Composition selon la revendication 1, dans laquelle les vésicules lipidiques ont été incorporées sous forme d'un concentrat obtenu par centrifugation.

3. Composition selon la revendication 2, comprenant dans une phase aqueuse jusqu'à 30% en poids du concentrat obtenu par centrifugation.

## Claims

1. Cosmetic or pharmaceutical composition for topical use comprising lipidic vesicles obtained by crushing oil-yielding grains, emulsification in an aqueous phase and filtration.

2. Composition according to claim 1 in which the lipidic vesicles have been incorporated in the form of a concentrate obtained by centrifuging.

3. Composition according to claim 2 comprising in an aqueous phase up to 30% by weight of the concentrate obtained by centrifuging.

## Patentansprüche

1. Kosmetische oder pharmazeutische Zusammensetzung zur topischen Anwendung, umfassend Lipid-Vesikel, erhalten durch Zerquetschen von ölhaltigen Körnern, Emulgieren in einer wäßrigen Phase und Filtrieren.

2. Zusammensetzung nach Anspruch 1, in die die Lipid-Vesikel in der Form eines durch Zentrifugieren erhaltenen Konzentrats eingearbeitet wurden.

3. Zusammensetzung nach Anspruch 2, umfassend in einer wäßrigen Phase bis zu 30 Gew.-% des durch Zentrifugieren erhaltenen Konzentrats.
